# EUROPEAN PATENT APPLICATION

(11) **EP 3 910 030 A1**
(43) Date of publication of application: **17.11.2021**
(21) Application number: 20173972.9
(22) Date of filing: 11.05.2020
(51) Int. Cl.: C09B 1/00, C09B 67/42, G01N 33/58, C07F 5/02

(54) **BIOINSPIRED FLUORESCENT STAINS FOR REVERSIBLE LABELLING OF THE ENDOPLASMIC RETICULUM AND FOR FURTHER ANALYTICAL APPLICATIONS**

(71) Applicant: Technische Universität München, 80333 München (DE); Deutsches Krebsforschungszentrum, 69120 Heidelberg (DE)
(72) Inventor: Delius, Judith, 85354 Freising (DE); Hofmann, Thomas, 85375 Neufahrn (DE); Kallenberger, Stefan, 69126 Heidelberg (DE)
(74) Representative: Moré, Solveig Helga

(57) **Abstract**

The present invention relates to the field of fluorescent compounds and dyes. In particular, it provides the use of a polyphenol, such as quercetin or luteolin, and a diaryl borinic compound, e.g., 2-aminoethoxydiphenyl borate (also referred to as 2-APB, diphenylborinic acid 2-aminoethyl ester, DPBA or Neu's reagent), in combination with an agent capable of providing an aprotic environment selected from the group consisting of the endoplasmic reticulum (ER) of an animal cell, a protein, a polypeptide, and a solid, preferably, crystalline phase of a complex formed by the polyphenol and the diaryl borinic compound, for generating an increased fluorescence. The invention teaches the use of a polyphenol and a diaryl borinic compound for selective fluorescent staining of the endoplasmic reticulum (ER) of an animal cell and a corresponding method as well as kits useful to this end. Advantageously, the ER can be reversibly stained, and, depending on the selection of the polyphenol, different wavelengths of fluorescence can be obtained. Also provided is the use of a polyphenol, a diaryl borinic compound, and an agent capable of providing an aprotic environment, e.g., a protein, for determining the proton-donating ability of a solvent, or determining the presence and/or concentration of the agent, the polyphenol or the diaryl borinic compound. Complexes of the invention, e.g., in solid or crystalline form, may also be used as fluorescent dyes or paints, or for providing light, e.g., in a display.

## Description

The present invention relates to the field of fluorescent compounds and dyes. In particular, it provides the use of a polyphenol, such as quercetin or luteolin, and a diaryl borinic compound, e.g., 2-aminoethoxydiphenyl borate (also referred to as 2-APB, diphenylborinic acid 2-aminoethyl ester, DPBA or Neu's reagent), in combination with an agent capable of providing an aprotic environment selected from the group consisting of the endoplasmic reticulum (ER) of an animal cell, a protein, and a solid, preferably, crystalline phase of a complex formed by the polyphenol and the diaryl borinic compound, for generating an increased fluorescence. The invention teaches the use of a polyphenol and a diaryl borinic compound for selective fluorescent staining of the endoplasmic reticulum (ER) of an animal cell and a corresponding method as well as kits useful to this end. Advantageously, the ER can be reversibly stained, and, depending on the selection of the polyphenol, different wavelengths of fluorescence can be obtained. Also provided is the use of a polyphenol, a diaryl borinic compound, and an agent capable of providing an aprotic environment, e.g., a protein, for determining the proton-donating ability of a solvent, or determining the presence and/or concentration of the agent, the polyphenol or the diaryl borinic compound. Complexes of the invention, e.g., in solid or crystalline form, may also be used as fluorescent dyes or paints, or for providing light, e.g., in a display.

A fluorophore (or fluorochrome, similarly to a chromophore) is a fluorescent chemical compound that can re-emit light upon light excitation. Fluorophores typically contain several combined aromatic groups, or planar or cyclic molecules with several conjugated π-bonds.

Polyphenols, i.e., aromatic compounds comprising two or more hydroxyl groups that are directly coupled to an aromatic ring, are widely distributed in fruit and vegetables, protect the plants from damage by UV radiation, provide antibiotic resistance to pathogens, and have antifeedant effects. As part of the human diet, polyphenols can reduce the risk of degenerative diseases, such as cancer or cardiovascular disorders. Although potential cellular targets have been identified for these chemopreventive agents, their definite biological mode of action is still obscure.

Quercetin has been described to activate or inhibit the biological activities of a number of proteins (Feitelson et al., Seminars in Cancer Biology 35: Suppl. S25-54). For example, quercetin is a non-specific protein kinase enzyme inhibitor (Williams et al., 2004. Free Radical Biology & Medicine. 36 (7): 838-49; Russo et al., 2014, Cancer Treatment and Research. 159: 185-205). It has been reported to bind to the kinase-RNase domain of the ER stress-induced kinase endonclease IRE1 (Wiseman etal., 2010, Mol Cell. 38(2):291-304). Quercetin has also been reported to have estrogenic activities by activating estrogen receptors (Moutsatsou 2007, Hormones (Athens, Greece). 6 (3): 173-93). In human breast cancer cell lines, quercetin has also been found to act as an agonist of the G protein-coupled estrogen receptor (GPER) (Maggiolini et al. 2004. The Journal of Biological Chemistry. 279 (26): 27008-16).

Polyphenols, e.g., flavonoids such as quercetin, are known to have fluorescent characteristics (e.g., Simkovitch et al., 2015, J.Phys. Chem. B 119:10244-10251; Momic et al., 2007, Collect. Czech Chem. Commun. 72(11): 1447-1460). For example, quercetin, like most flavonoids, is poorly fluorescent in aqueous solutions, but exhibits an increased fluorescence when bound to specific probes such as 2-APB (Filippi et al., 2015, FEBS Open Bio 5: 748-752). The stronger fluorescence in combination with the diaryl borinic compound 2-APB, which increases the sensitivity of flavonoid detection by the formation of coordination complexes with 3-hydroxyflavones (Lee et al., 2014, Food Sci. Biotechnol. 23(1): 75-79; Cordoba at al., 2015, ACS Appl. Mater. Interfaces 7:16857-16864), is exploited in the art. For example, Lee at al. teach a method for quantification of cellular flavonoid levels in mammalian cells using quercetin and 2-APB, e.g., based on fluorescence-activated cell sorting (FACS), and also stain quercetin in the cells with 2-APB. Filippi et al. use 2-APB to monitor flavonoid uptake across plant cell membranes and discuss that nucleus and cytoplasm of the plant cells are stained.

In general, fluorophores are sometimes used alone, as a tracer in fluids, as a dye for staining of certain structures, as a substrate of enzymes, or as a probe or indicator (when its fluorescence is affected by environmental aspects such as polarity or ions). Often, they are covalently bonded to a macromolecule, serving as a marker (or dye, or tag, or reporter) for affine or bioactive reagents such as antibodies, peptides, or nucleic acids. Fluorophores are notably used to stain tissues, cells, or materials in a variety of analytical methods, i.e., fluorescent imaging and spectroscopy.

The ability to distinguish and identify specific subcellular compartments of cells is essential to understand organelle function, biogenesis, and maintenance within cells and to defining protein trafficking pathways. Numerous fluorescent dyes that are capable of staining different cell compartments, e.g., the nucleus, cell membrane, ER, Golgi complex, and mitochondria, are known in the art. For example, selective and non-selective fluorescent probes for staining the ER are reviewed by Colston et al., 2003 (Biotechnic & Histochemistry 78(6):323-332). Colston et al. discuss that the basis for selectivity of the fluorochromes routinely used to visualize the ER in live cells remains obscure. The most widely used membrane-permeable fluorescent dyes for visualisation of the ER are DiOC₆(3), DiOC₅(3) and hexylrhodamine B or, more recently, ER-tracker blue-white DPX/ER tracker. Some of these dyes are taught to be selective, but not specific for the ER, i.e., they may also stain other cell structures such as mitochondria. Non-routine or inconsistent ER stains include cresyl fast violet, DilC₁(3) and related dyes, new methylene blue CI52030, purpurin CI75410 or quinazurin, as well as TMR esters.

In light of the state of the art, the inventors solved the problem of providing a novel fluorescent method for staining of the ER, which has the advantage of allowing for a reversible staining, and related analytical methods and uses. This problem is solved by the present invention, in particular, as presented in the claims.

The invention provides a use of a polyphenol and a diaryl borinic compound in combination with an agent capable of providing an aprotic environment selected from the group consisting of
a) the endoplasmic reticulum (ER) of an animal cell,
b) a protein, and
c) a solid, e.g., a crystalline phase of a complex formed by the polyphenol and the diaryl borinic compound,
for providing an increased fluorescence, wherein the fluorescence is increased by a factor of at least 2 in the presence of the agent compared to the absence of the agent. The fluorescence is preferably increased by a factor of at least 5, at least 10 or at least 20.

The increase of fluorescence induced by the agent capable of providing an aprotic environment allows for various advantageous analytical applications. In particular, the invention presents a series of plant-derived fluorescent switch-on probes and their application, in particular, in live-cell imaging, in the detection of even low abundant proteins, and pH measurements. The fluorogens are polyphenols, typically plant-based polyphenols, that are coordinated to the diaryl borinic compounds such as 2-APB. The inventors discovered that a rapid and significant fluorescence enhancement of the weakly emissive aqueous solutions of the complexes of polyphenol and 2-APB emerges when they interact with agents capable of providing an aprotic environment, e.g., a subcellular compartment like the endoplasmic reticulum, with proteins or aprotic solvents. The inventors demonstrated that the staining of the ER of an animal cell is selective and reversible and has further advantages due to different options for selecting a polyphenol leading to a desired excitation and emission wavelength.

### ER staining

Accordingly, in a preferred embodiment, the invention provides use of a polyphenol and a diaryl borinic compound for selective fluorescence staining of the endoplasmic reticulum (ER), in particular, the ER of an animal cell. This allows for imaging of the cell and its sub-structures, in particular, the ER, i.e., for analysis of the ER in the cells. The invention also provides a method for selective fluorescence staining of the endoplasmic reticulum, in particular, the ER of an animal cell, comprising contacting the cell with a polyphenol and a diaryl borinic compound, wherein the analysis is carried out in the presence of the diaryl borinic compound.

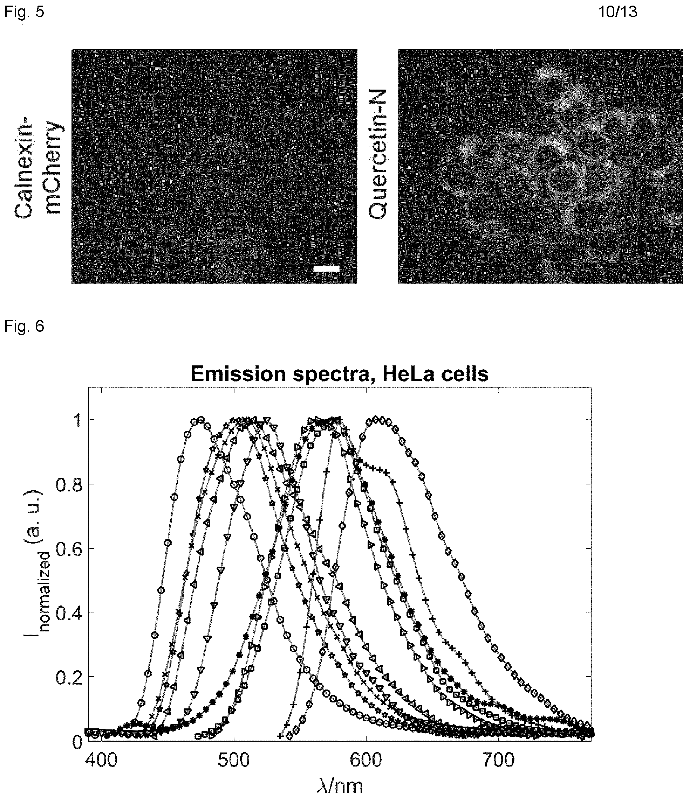

The ER is a type of organelle found in eukaryotic cells that forms an interconnected network of flattened, membrane-enclosed sacs or tube-like structures known as cisternae. The membranes of the ER are continuous with the outer nuclear membrane. The ER occurs in most eukaryotic cells, but is absent from red blood cells and spermatozoa. In light of the prior art characterizing a combination of polyphenols and the diarylborinic compound 2-APB as staining the nucleus and cytoplasm of the plant cells (Filippi et al., 2015), it is highly surprising that a selective staining of the ER can be found with these agents in animal cells. Thus, preferably, the ER is stained in animal cells, e.g., mammalian cells. Cells can be e.g. human cells or murine cells. Cells can be primary cells or cell lines. The inventors could show that there is a strong correlation between staining with a polyphenol and a diaryl borinic compound and the ER marker calnexin-mCherry or calnexin-mGFP, proving a co-localization and selective ER staining (Fig. 1AB and 5). In contrast to these latter ER markers, which are typically transiently expressed, the method of the present invention can also be used to stain the ER in non-transfectable cells. The inventors noted that, while the ER is not stained by calnexin-mCherry or calnexin-mGFP in every cell of a composition comprising a plurality of cells, the ER is stained in most, or all of the cells upon use of the method of the invention.

The cells can be fixed or non-fixed cells. For example, the cells can be living cells.

The cells may be contacted with the diaryl borinic compound and the polyphenol separately, in any order. Preferably, there is no, or only a small delay between the contacting with the agents, e.g., less than 5 minutes, less than 3 minutes, or less than 1 minute, preferably, less than 20 seconds. If the time between these contacts is long enough to allow for diffusion of the molecules out of the cells, the sensitivity is lower, or staining may even be absent, e.g., if no diaryl borinic compound is present for more about 10 minutes. In contrast, the complex is stable in the cells as long as the diaryl borinic acid is present.

Accordingly, in a preferred embodiment, the ER of the cells is stained by contacting them with a mixture of the diaryl borinic compound and the polyphenol, e.g., for at least 10 seconds or at least one minute, such as 1-30 minutes, preferably, 2-15 minutes, 2-10 minutes or 3-5 minutes. Three minutes of staining has been shown to be sufficient to achieve full fluorescence of the ER. The ER of living cells is preferably stained at 37°C. Fixed cells are preferably stained at room temperature and can then be stored, e.g., in a refrigerator at 4°C, for longer times, e.g., over night, for days or weeks.

Alternatively, the staining of the ER may be performed by sequentially contacting the cells with the polyphenol, e.g., for 1-30 minutes, preferably, 2-15 minutes, 2-10 minutes or 3-5 minutes, followed by contacting the cells with the diaryl borinic compound, e.g. 2-APB, e.g., for 10 seconds - 10 minutes, preferably, for 30 seconds - 3 minutes. Living cells are preferably stained at 37°C and fixed cells are preferably stained at room temperature. In case, the polyphenol is removed from the medium, in presence of 2-APB, the fluorescence staining of the ER remains for at least several hours, e.g., for weeks.

The contacting with the diaryl borinic compound may be at a concentration of about 1-1000 µmol L⁻¹, preferably, 5-50 µmol L⁻¹ or 8-10 µmol L⁻¹ of the the diaryl borinic compound, e.g., 2-APB. The concentration during the analysis, and, preferably, during washing step(s) are typically is in the same range.

The contacting with the polyphenol may be at a concentration of about 0.1-1000 µmol L⁻¹, preferably, 1-100 µmol L⁻¹ or 5-10 µmol L⁻¹ of the polyphenol. As discussed before, mixtures of both diaryl borinic compound and polyphenol may be used for staining.

For cellular imaging applications, both reagents are typically in an aqueous solution (either together, or separately). If the cells are living cells, the solution is preferably non-toxic. The aqueous solution may be a buffer, e.g., PBS, or a cell culture medium. It preferably does not comprise any compounds that produce non-targeted fluorescence that may impede imaging.

The inventors could show that, while detection of the ER staining is possible without washing, the image contrast is increased by washing with a solution comprising the diaryl borinic compound, but not the polyphenol. The washing and analysis are preferably carried out in the presence of the diaryl borinic compound, because, in the absence thereof, the staining rapidly vanishes.

Preferably, the staining of the ER is performed by contacting the cells with the polyphenol, preferably, with a mixture of the polyphenol and the diaryl borinic compound, and then washing the cells with the diaryl borinic compound, wherein the staining is analysed, e.g., by fluorescence microscopy, in the presence of the diaryl borinic compound.

The spectroscopic properties of the complexes of polyphenol, diaryl borinic agent and agents capable of providing an aprotic environment, e.g., the ER, formed according to the invention are tunable depending on the molecular structure of the polyphenol. This enables multicolor experiments. The most suitable complexation partners provided, i.e., quercetin, luteolin, esculetin, alizarin, and purpurin, allow for microscopic imaging with standard laser wavelengths and other common excitation sources. The corresponding dyes feature relatively large Stokes shifts (1800-4100 cm⁻¹), preventing signal overlap between the excitation and emission channels.

The option to synthesize and apply further structural derivatives as complexation agents for the diaryl borinic compound opens up an even broader spectral range of excitation and emission wavelengths.

Optionally, the cells may at the same time, or sequentially in any order, be stained with one or more reagents (e.g., a nuclear staining reagent (e.g., DAPI or Hoechst), a mitochondrial staining reagent, a lysosomal staining reagent, a cytosomal staining reagent, a membrane staining reagent, an agent suitable for staining the cytoskeleton such as phalloidin, an antibody to a structure of interest etc.) suitable for imaging of other sub-structures, preferably, with a staining reagent leading to fluorescence that can be distinctly measured or detected compared to ER stain of the invention. The colour of the ER stain of the invention may be selected by selection of the polyphenol, as described herein. Of course, the cell may already comprise fluorescent components, such as fluorescent proteins, e.g., fusion proteins with proteins, such as GFP, YFP, and RFP.

The contact with the cell may be in any form, e.g., in a cell culture plate, a tube or on a slide suitable for imaging, e.g., a cytospin, a drop of cells in culture medium or buffer, or a tissue section. Contacting may be at any temperature suitable for maintenance of cells, e.g., 0-37°C, on ice, at about 4°C, at room temperature (20-25°C) or at 37°C.

Advantageously, in the use or method of the invention, the staining of the ER is reversible. The ER is stained in the presence of the diaryl borinic compound and the ER is not stained in the absence of the diaryl borinic compound. Thus, for ER imaging, the cells are maintained in the presence of the diaryl borinic compound. If, e.g., for subsequent imaging of any other compound, it is desired to erase the fluorescent staining of the ER, the cells may be washed with buffer or cell culture medium not containing a diaryl borinic compound.

The lack of awareness of the reversibility of the staining of polyphenols with 2-APB, and, consequently, the implementation of washing steps in the absence of 2-APB, may account for the failure in the prior art to determine that a complex of the diaryl borinic compound and polyphenols of the invention are suitable for selectively staining the ER.

In one aspect of the invention, the reagents, i.e., diaryl borinic compound and polyphenol, separately or in a mixture, may be sprayed on the sample comprising the cells to be analysed, which may already be present on a support, e.g., a slide suitable for fluorescence microscopy. For washing and analysis, a further reagent comprising the diaryl borinic compound, but not the polyphenol, may be used, e.g., sprayed on the sample. If erasure of the ER stain of the invention is desired, a reagent not comprising the diaryl borinic compound, and typically, not comprising the polyphenol, may be administered, e.g., again, by spraying it on the cells.

In another aspect, the reagents, i.e, the diaryl borinic compound and the polyphenol, separately or in a mixture, may be administered to the cells or the sample comprising the cells to be analysed by means of a brush or a pen capable of dispensing the reagents. For example, a brush attached to the screw cap of a bottle or a pen similar to a text marker pen that comprise one, or preferably both reagents, may be used for staining. If a reversal of staining is desired, the cells may be washed in the absence of the diaryl borinic compound, e.g., by rinsing or spraying with a solution that does not contain the diaryl borinic compound. Kits and combinations of such pens are also provided, e.g., a staining spray, brush attached to a screw cap bottle or pen in combination with a washing solution.

The method of the invention for staining the ER may be used for different purposes, e.g., in the context of research, for diagnostic purposes, to detect specific cell types on slides with tissue sections or smear samples, to distinguish between cells with or without ER, to characterize the sarcoplasmic reticulum of cardiomyocytes, to stain cells for fluorescence-activated cell sorting (FACS), in single-cell analysis, pathology or for screening of potential drugs, e.g., drugs affecting characteristics of the ER in cells, e.g., human cells. In applications such as FACS, it is advantageous that the fluorescent staining can be easily removed by washing with a fluid that does not contain the diaryl borinic compound to enable subsequent staining of different cellular structures.

It is a decisive advantage that the diaryl borinic compounds 2-APB is a low cost bulk chemical (e.g. 50 g = 244 Euro, Carl Roth, Karlsruhe, Germany) compared to commercial organelle stains for live-cell imaging (e.g. ER-Tracker™ Green, BODIPY® FL Glibenclamide, 100 µg = 434 Euro, Life Technologies GmbH, Darmstadt, Germany) with a price factor of one million. Therefore, the invention provides 2-APB and polyphenols, as described herein, as cheap alternatives to ER-Tracker™ dyes. The ease of dye preparation, the fast staining and destaining kinetics, and the cost-effectiveness make the new polyphenol-based dyes, which are designated ERasers, ideal fluorescent labels for the ER and other versatile applications in biochemistry.

### Analytical uses

The inventors could further show that the polyphenol and the diaryl borinic compound do not only have an increased fluorescence upon binding to the ER of an animal cell, but also, in case they are associated with other agents capable of providing an aprotic - or hydrophobic - environment.

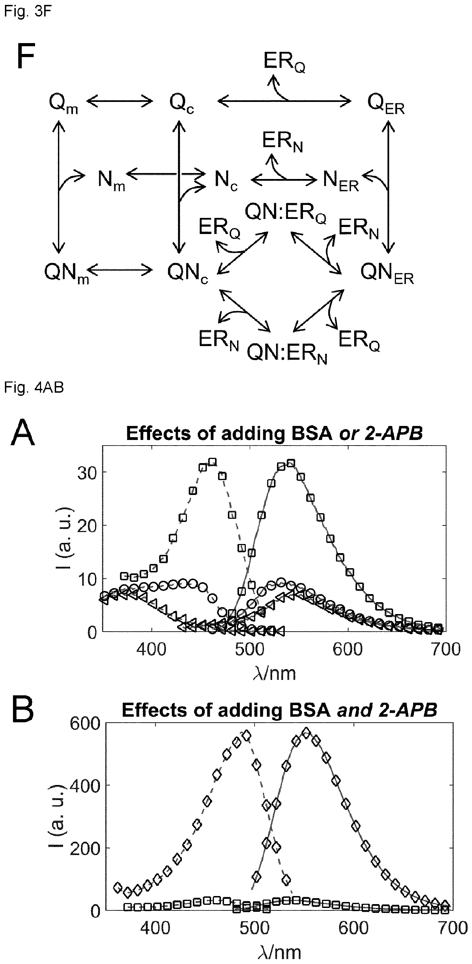

For example, in the presence of proteins such as BSA, there was a markedly increased fluorescence (cf. Fig. 4B, Fig. 7, Fig. 8, Fig. 9). The fluorescence signal linearly increased with the protein concentration. Accordingly, the invention also provides use of a polyphenol and a diaryl borinic compound for determining the presence and/or concentration of a protein in a composition, e.g., on a blotting membrane. The composition may also be a solution.

The protein may be, e.g., albumin, for example BSA or human serum albumin. It may also be another protein, such as insulin, egg white proteins, ovalbumin, ovotransferrin, ovomucoid, ovoglobulin, an immunoglobulin, e.g., IgG, IgM, IgE, IgD, or IgA, a human saliva protein. Due to the low price and common availability of albumins, use of albumins such as BSA is advantageous. The term protein includes polypeptides.

While the detection of specific proteins is not specific for such proteins, specific proteins, e.g., insulin, can be sensitively detected and/or quantified after separation or isolation.

The inventors found that the sensitivity of detection of all of these proteins was higher than with other proteins such as lysozyme. Without intending to be bound by the theory, it is believed that the sensitivity, or the increase in fluorescence mediated by the protein, depends on the capability of the protein to bind the complex of the polyphenol and the diaryl borinic compound in an aprotic environment, e.g. an aprotic binding pocket. The sensitivity of protein detection for different proteins can be easily tested by a titration of said protein with the polyphenols while determining fluorescence at a suitable wavelength. Preferably, for a quantitative determination, the fluorescence in the sample is compared to the fluorescence of a standard curve prepared with the same protein. Preferably, in a sample in which the concentration of protein is to be determined, there is only one kind of protein, which is known beforehand to allow for preparation of a suitable standard curve. Alternatively, mixtures can be used, wherein the standard preferably is a corresponding mixture. The pH is preferably kept constant for the analysis of proteins within the scope of this invention.

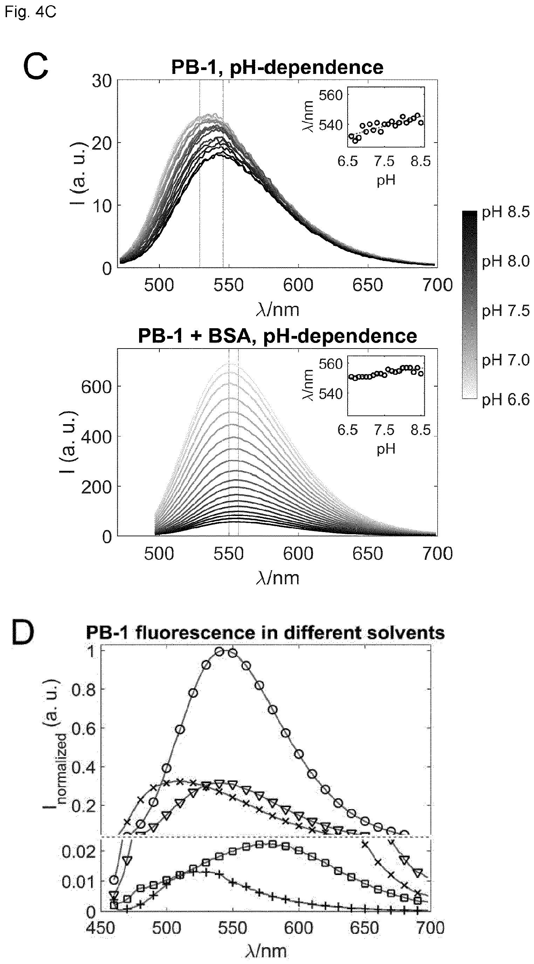

While it was known in the art that the pH of a solution affects the fluorescence of polyphenols, the inventors further found that in the presence of protein, e.g., BSA or the other proteins defined herein, the location of the fluorescence maximum was rather independent from the pH of the solution, but the fluorescence intensity was significantly more sensitive to the solution pH than without protein (Fig. 4C, bottom). This suggests that pH-induced conformational changes of the protein cause the strong pH dependence of the fluorescence strength of the ternary system or protein, diaryl borinic compound and polyphenol. As the fluorescence intensity was correlated with the pH of the solution, combinations of polyphenols, diaryl borinic compound and protein, e.g., BSA, may be used as a pH indicator.

Furthermore, a pronounced change in the fluorescence intensity depending on the proton-donating ability of the solvent was observed by the inventors. In aprotic solvents, e.g., DMSO, dichloromethane, and particularly in acetonitrile, in which flavonoids remain unionized, the fluorescence intensity was extraordinarily high, whereas it drastically declined in protic solvents, such as MeOH or water (Fig. 4D). Thus, polyphenols and diaryl borinic compound may also be used to determine the proton-donating ability of a solvent, optionally, in the presence of a protein such as BSA. The invention also provides use of a polyphenol, a diaryl borinic compound, and, optionally, a protein for determining the presence and/or concentration of an aprotic solvent.

In another aspect, the increased fluorescence of the ternary complex of a polyphenol, a diaryl borinic compound, and an agent capable of providing an aprotic environment, e.g., a protein such as BSA may also be used for determining the presence and/or concentration of the other components of said complex, namely, the polyphenol or the diaryl borinic compound.

Thus, the invention provides use of a diaryl borinic compound, and an agent capable of providing an aprotic environment, preferably, a protein such as BSA for determining the presence and/or concentration of the polyphenol. This method has the advantage of being very sensitive in comparison to known methods of determining the presence or concentration of polyphenols in the absence of protein. Preferably, the type of polyphenol is known beforehand and a calibration curve is used for quantitation. Alternatively, the polyphenol can be determined, e.g. by comparing its fluorescence emission spectrum with the spectra of polyphenols characterized before.

The invention also provides use of a polyphenol and an agent capable of providing an aprotic environment, preferably, a protein such as BSA for determining the presence and/or concentration of the diaryl borinic compound, preferably, 2-APB.

Corresponding methods are also disclosed, wherein the sample is contacted with
- the polyphenol and the diaryl borinic compound for analysis of the presence and/or concentration of a protein or an aprotic solvent in the sample, or
- the polyphenol, a protein and the diaryl borinic compound for determining the pH of an aqueous solution sample, or
- the diaryl borinic compound and an agent capable of providing an aprotic environment, preferably, a protein such as BSA, for determining the presence and/or concentration of a polyphenol in the sample, or
- the polyphenol and an agent capable of providing an aprotic environment, preferably, a protein such as BSA, for determining the presence and/or concentration of a diaryl borinic compound in the sample.

The fluorescence of the sample is then determined at a suitable wavelength, dependent on the selected polyphenol.

For determining the concentration, fluorescence is typically compared with the fluorescence of a suitable standard, e.g., in a calibration curve.

### Fluorescent paints and solids

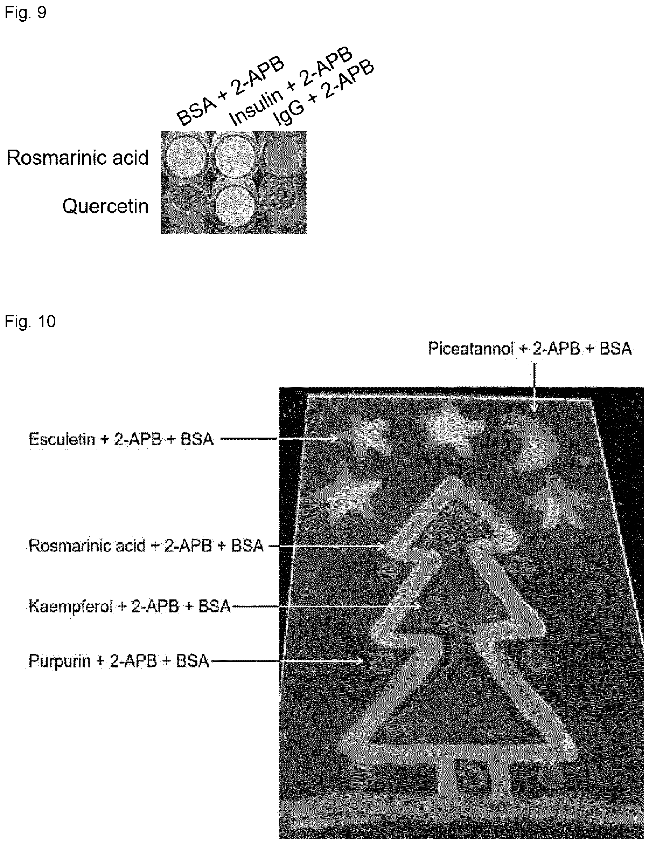

The inventors have found that the polyphenol and diaryl borinic compound, optional, in combination with a protein, can also be used as paints (cf. Fig. 10) or for providing light, e.g., in solid form, optionally, in crystalline form. In particular, they noted that in a crystalline phase of a complex formed by the polyphenol and the diaryl borinic compound, the fluorescence is also significantly increased. Accordingly, a crystalline phase of a complex formed by the polyphenol and the diaryl borinic compound can be used for providing light, e.g., in a light emitting element such as a display, or light emitting device. Such crystals have a strong fluorescence and a high quantum yield.

Crystals may be prepared according to the invention by providing a supersaturated solution of 2-APB and the polyphenol until crystallization sets in (Fig. 11AB). Supersaturation can be achieved, e.g., by the vapor diffusion method, or evaporation of the volatile solvent, such as methanol or ethanol. As the solvent slowly evaporates, the solution becomes supersaturated and colourful crystals form, which grow in size over time. According to x-ray structure analysis, the crystals mainly consist of 2-APB doped with traces of polyphenol. Crystals are highly fluorescent, when excited at the appropriate wavelength.

A fluorescent paint can also be prepared according to the invention, as the inventors have found that a mixture of a polyphenol, a diaryl borinic compound such as 2-APB and a protein, e.g., BSA or any of the other proteins cited herein, can be used as a fluorescent paint.

Such paint can be liquid, wet or dry. For example, a solution thereof may be administered to a support, e.g., a glass plate, paper, or a wall. The paint continues to provide fluorescence after drying. The recited components can be further mixed with other components, e.g., increasing the stability or increasing adhesion to a specific support. The colour of the paint depends on the selection of the polyphenol, so different colours, e.g., blue, red, green, cyan, or orange can be prepared. Mixtures of polyphenols may lead to other colours, e.g., white. Such paints may provide, e.g., fluorescence when excited with light of a suitable wavelength, e.g., UV light.

If long-term fluorescence is desired, it may be useful to reduce or prevent exposure to oxygen to increase the stability of the polyphenols.

A mixture of a polyphenol, a diaryl borinic compound such as 2-APB and a protein, e.g., BSA or any of the other proteins cited herein can also be embedded into a polymer and used as a fluorescent foil or as a display.

### The diaryl borinic compound

Throughout the invention, the diaryl borinic compound preferably is a compound of formula 10, 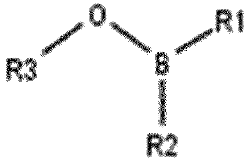 wherein R1 and R2 are independently from one another selected from non-substituted aryl, substituted aryl, non-substituted heteroaryl and substituted heteroaryl, preferably, non-substituted phenyl,
wherein R3 is C₂-C₁₀ alkylamino, C₁-C₁₀ alkyl, C₂-C₁₀ alkoxy, Ce-Cio aryl, C₅-C₁₀ heteroaryl or C₇-C₁₀ aralkyl, preferably, C₂-C₁₀ alkylamino.

R1 and R2 may be the same or different. Aryl is any functional group or substituent derived from an aromatic ring, usually an aromatic hydrocarbon, such as phenyl, naphthyl, tolyl, xylyl. Heteroaryl may be, e.g., furyl, thienyl, pyrrolyl, or pyridyl. Substitutions may be, e.g., with one or more C₁-C₆ alkyl such as methyl or ethyl, or with -OH, preferably, with a C₁-C₆ alkyl group.

Preferably, R1 and/or R2 are phenyl, e.g., both. Good results have been obtained with unsubstituted phenyl. Accordingly, preferably, the diaryl borinic compound is a diphenyl borinic compound. Alternatively, the phenyl may be substituted, e.g., with one or more C₁-C₆ alkyl such as methyl or ethyl, or with -OH, preferably, with a C₁-C₆ alkyl group.

R3 may be C₂-C₁₀ alkylamino, C₁-C₁₀ alkyl, C₂-C₁₀ alkoxy, C₆-C₁₀ aryl, C₆-C₁₀ heteroaryl or C₇-C₁₀ aralkyl. For example, it may be C₂-C₆ alkylamino, C₁-C₆ alkyl, C₂-C₆ alkoxy, C₆ aryl, C₅-C₆ heteroaryl or C₇ aralkyl. C₂ alkylamino, C₂ alkyl, C₂ alkoxy are of a preferred size. Preferably, R3 is C₂-C₁₀ alkylamino, e.g., C₂-C₆ alkylamino or C₂-C₃ alkylamino. Preferably, throughout the invention, the diaryl borinic compound is 2-aminoethoxydiphenyl borate.

### The polyphenol

Polyphenols are aromatic compounds comprising two or more hydroxyl groups directly bound to an aromatic ring. Natural polyphenols occur in plants as phytochemicals. They are bioactive substances such as pigments, aromatic agents and tannins and may protect the plant from predators or attract, e.g., insects. Polyphenols can also be basic building blocks for biopolymers such as lignin or suberin.

The inventors have found that a bright fluorescence is evoked in the context of the invention if the polyphenol comprises a catechol, a pyrogallol and/or a keto-enol moiety or a derivative thereof, wherein the catechol, pyrogallol and/or keto-enol groups of said moieties are substituents of a π-conjugated scaffold comprising at least 6 delocalized electrons. In particular, for use in staining or imaging the ER according to the invention, the π-conjugated scaffold preferably comprises at least 9 delocalized electrons, e.g., 9-16 delocalized electrons, as these polyphenols provide a brighter staining of the ER in a convenient wavelength range.

The polyphenol may be a natural, plant-derived polyphenol, or a synthetic or semi-synthetic polyphenol, or a mixture of such polyphenols, e.g., derivable from a plant extract. The polyphenol may have at least one binding site for the diaryl borinic compound, e.g. at least one pyrocatechol or pyrogallol structural element or repeating phenolic moieties of pyrocatechol or pyrogallol (as in hydrolyzable and nonhydrolyzable condensed tannins).

Preferably, it is a naturally occurring polyphenol. The polyphenol may be, e.g.,
a) a flavonoid selected from the group comprising flavonole, hydroxyflavone, flavan-3-ol, anthocyanidine, flavanone, isoflavone,
b) a hydroxyantraquinone,
c) a benzopyrone,
d) a stilbenoid,
e) a chalcone,
f) a curcuminoid,
g) a hydroxycinnamic acid,
h) a hydroxybenzoic acid,
i) pyrocatechol,
or a derivative thereof. A derivative may be, e.g. an ether, a glycoside or an ester of the polyphenol, for example, a glycoside of the flavonoid.

The inventors successfully detected an improved fluorescence according to the invention with the polyphenols quercetin, piceatannol, curcumin, rosmarinic acid, luteolin, myricetin, esculetin, purpurin, alizarin, kaempferol, epigallocatechin gallate, caffeic acid, or derivatives thereof, wherein the derivative optionally is a glycoside. For example, quercetin-3-O-glucorhamnosid or corresponding derivatives of other flavonoids may be employed. Preferably, the polyphenol is quercetin or luteolin. Quercetin is preferred, because it is easily available at relatively low cost. On the other hand, luteolin complexes have a slightly higher fluorescence intensity.

Optionally, the π-conjugated scaffold of the polyphenol comprises two or three aromatic or heteroaromatic rings, wherein at least two of said rings are fused and wherein the polyphenol comprises two, but not three vicinal hydroxyl groups. Such polyphenols are particularly suitable for staining the ER. A heteroaromatic ring preferably comprises only C and O.

The inventors have analysed the molecular structures of complexes formed between the polyphenol and the diaryl borinic compound. Preferably, the polyphenol and the diaryl borinic compound are capable of reacting to form a fluorogenic complex comprising the structural element of formula 11: 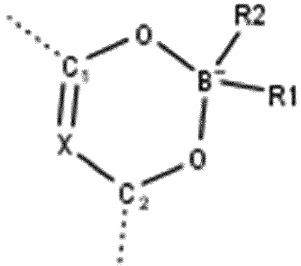 wherein R1 and R2 are independently selected from non-substituted aryl, substituted aryl, non-substituted heteroaryl and substituted heteroaryl, e.g., as defined above in the context of the diaryl borinic compound.
wherein X is absent or present,
and wherein, if X is absent, the double bond indicated between C₁ and X is between C₁ and C₂,
and wherein, if X is present, X is C,
wherein C₁ and C₂ and, if present, X, are part of an aromatic or heteroaromatic system, which system may be substituted or non-substituted,
wherein, preferably, X is absent.

In the structural element of formula 11, preferably, X is absent, and C₁ and C₂ are part of an aromatic or heteroaromatic ring system, which ring system may be substituted or non-substituted (formula 11a):

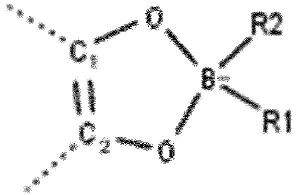

In the context of the invention, the term "complex" designates an association between two or three binding partners. The association can be covalent, e.g., as depicted in formula 11 or 11a, or it can be mediated by other forces, e.g., ionic binding, hydrophobic interactions or Van-der Waals forces.

### Products of the invention

The invention also provides specific products that are particularly useful for carrying out the methods of the invention.

In one aspect, the invention provides a kit suitable for the use or methods of the invention, particularly for staining the ER of animal cells, wherein the kit comprises
(i) a composition comprising (a) a polyphenol and, preferably, (b) a diaryl borinic compound,
(ii) a composition comprising the diaryl borinic compound.

The composition of (i) may be advantageously used, for example, for staining the ER of animal cells, as described herein. The composition of (ii) may be advantageously used, for example, for washing the sample stained with the composition of (i). The composition of (ii) does not comprise the polyphenol. The kit may comprise the components in a dry, e.g., lyophilised form, or in the form of a solution. Buffers or cell culture media, such as DMEM or RPMI, are preferred for analysis of live cells. HBSS (Hank's balanced salt solution) or PBS (phosphate buffered saline, pH 7.2-7.4) is a preferred buffer.

The kit optionally further comprises a protein, e.g., albumin, e.g., BSA or human albumin, or any of the other proteins described herein. The kit may alternatively or additionally comprise a composition, e.g., suitable for washing cells (iii) not comprising a diaryl borinic compound, which typically also does not comprise the polyphenol.

Such compositions (i), (ii) and/or (iii) may be in the form of sprays or pens capable of dispensing the respective compositions, e.g., as described above.

The invention also provides a kit, e.g., a kit as described above, comprising at least 2, preferably, 3 or 4 different polyphenols, each having, in complex with the diaryl borinic compound, an emission wavelength that can be differentiated from the emission wavelength of the other complex(es), wherein the kit optionally comprises a separate composition (i) for each polyphenol. For example, the kit of the invention may comprise a polyphenol emitting blue light, and a polyphenol emitting red or orange light. In a preferred embodiment, the kit comprises esculetin (bluish fluorescence), quercetin or luteolin (greenish fluorescence), and/or alizarin or purpurin (reddish fluorescence), preferably, a combination of three polyphenols emitting blue, green or red light, respectively. The different colours should be spectrally separable by standard sets of excitation and emission filters of optical microscopes, e.g. filters for DAPI, GFP, and RFP.

In one aspect, the invention also provides an isolated fluorescent complex comprising (a) a polyphenol, (b) a diaryl borinic compound, and (c) a protein, e.g., as described herein, for example, albumin. Also provided is a kit comprising (a), (b) and (c), wherein the complex is preferably obtainable by combining the components of the kit described herein.

For example, for the first time, the invention provides a kit, complex or composition, comprising (a) a polyphenol that is not a flavonoid and (b) a diaryl borinic compound, optionally, further comprising (c) a protein. The use of said diaryl borinic compound for analysing the presence and/or concentration of said polyphenol that is not a flavonoid, optionally, in the presence of (c) a protein, is also disclosed.

In another aspect, the invention provides a solid, optionally, crystalline form of a complex of (a) a polyphenol and (b) a diaryl borinic compound, wherein the complex optionally further comprises (c) a protein. As described herein, the solid complex of (a) the polyphenol and (b) the diaryl borinic compound is useful for providing light, e.g., in a light emitting device or display. The complex of polyphenol, diaryl borinic compund and protein may e.g., be used as a fluorescent paint, e.g., in wet or dry form. A solid support, e.g., a glass plate with the associated complex, which is capable of being illuminated with a suitable excitation wavelength, e.g., UV light, is also provided. The solid support with the paint may also be a sign or display.

### Figure legends

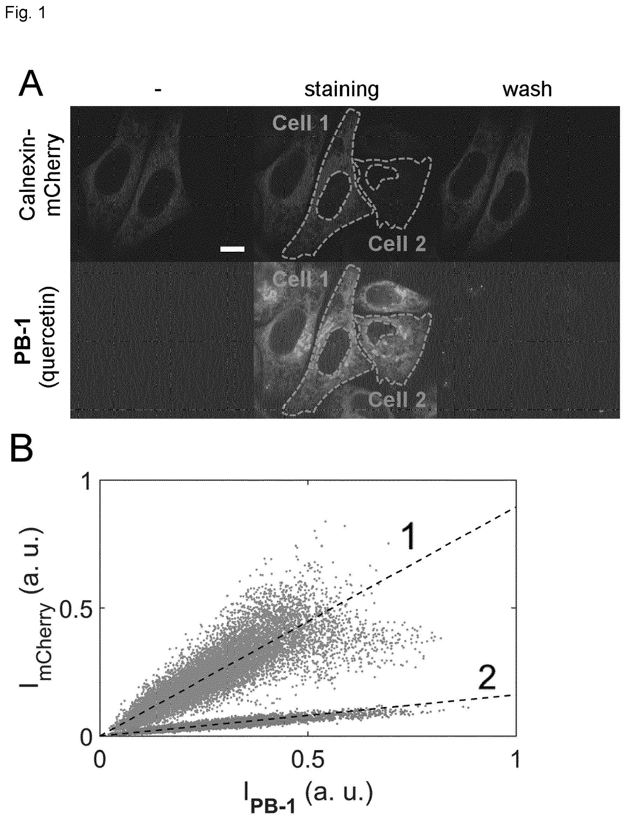

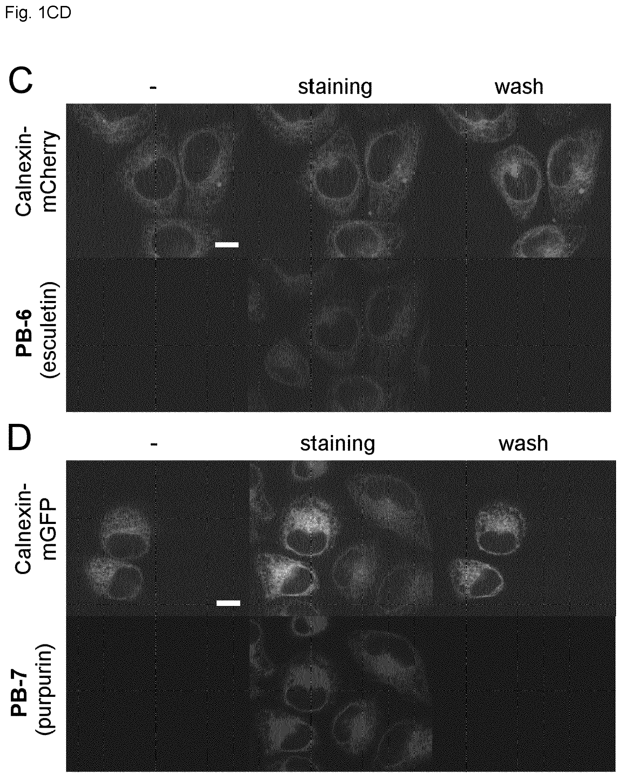

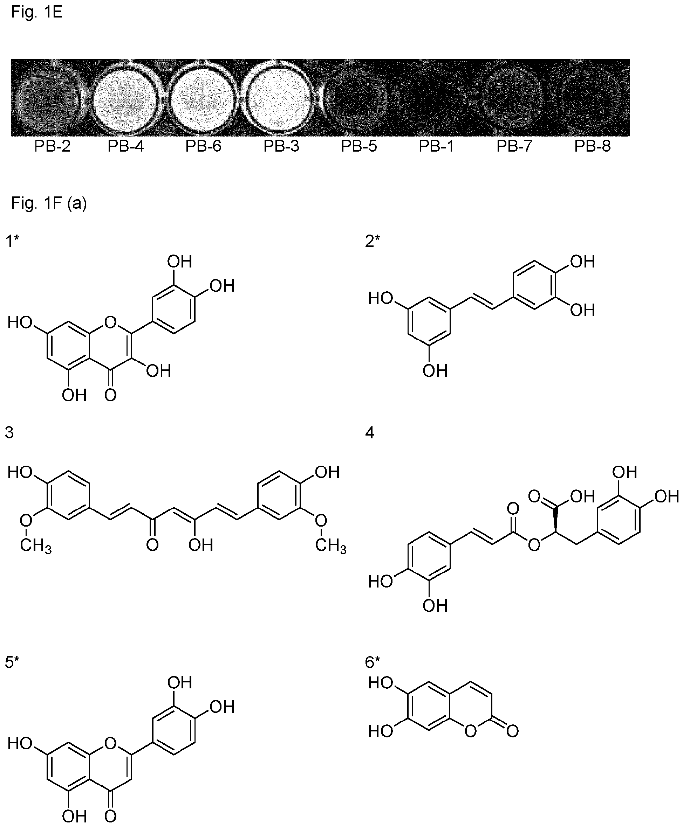

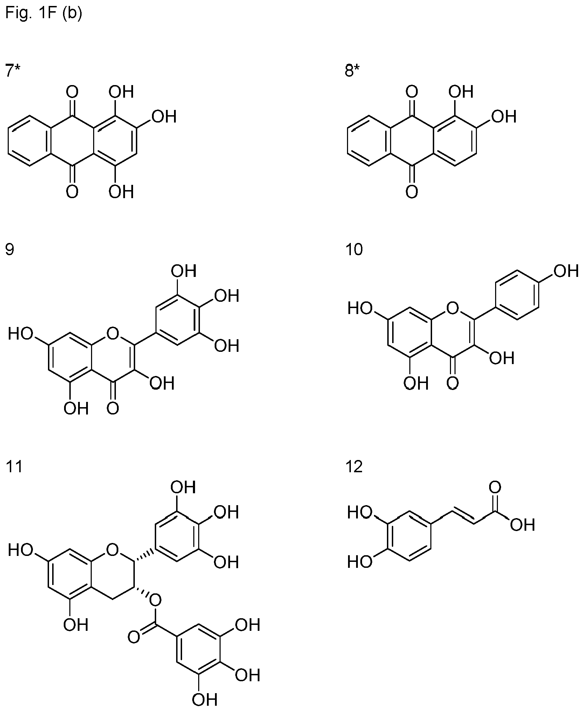

**Fig. 1****. (A) Reversible ER staining with PB-P** (**PB**=di**p**henyl**b**oran / diphenyl borinic compound)-(**P**=**p**olyphenol) **in HeLa cells.** Confocal microscopic images were taken prior to staining, after incubating with **PB-1** (quercetin, green fluorescence), and subsequent to washing with growth medium. Expression of the ER-marker calnexin-mCherry (red fluorescence) was partial (e.g., expression in cell 1 > cell 2). **(B)** Normalized fluorescence intensities of the red and green pixels within the cellular ROls marked in (A) are highly correlated, revealing co-localization of calnexin-mCherry and **PB-1.** Pixels for cell 1 (indicated by "1"), high mCherry expression, cluster around the upper line, while pixels for cell 2 (indicated by "2"), low mCherry expression, cluster around the lower line. **(C, D)** Reversible ER-staining with **PB-6** (esculetin, cyan fluorescence) or **PB-7** (purpurin, red fluorescence) according to the corresponding protocol used in (A). The ER was counterstained in (D) with calnexin-mGFP (red fluorescence) (scale bar: 10 µm).
   **(E)** The 2-APB complexes of the polyphenols piceatannol (**PB-2**), rosmarinic acid (**PB-4**), esculetin (**PB-6**), curcumin (**PB-3**), luteolin (**PB-5**), quercetin (**PB-1**), purpurin (**PB-7**), and alizarin (PB-8) are highly fluorescent in aprotic environments. Multiple color emission from blue **(PB-2),** cyan (**PB-3, PB-4, PB-6**), green (**PB-1, PB-5**) to orange (**PB-7**) and red (**PB-8**) of **PB-P** (in DMSO) results from excitation under ultraviolet light at 365 nm (**PB-1** and **PB-5** require *λ*ₑₓ=440 or 480 nm, respectively, to appear intensely fluorescent).
   **(F) Structures of the polyphenols** quercetin (**1***), piceatannol (**2**), curcumin (**3**), rosmarinic acid (**4**), luteolin (**5***), esculetin (**6***), purpurin (**7***), alizarin (**8***), myricetin (**9**), kaempferol (**10**), epigallocatechin gallate (**11**), caffeic acid (**12**). Preferred polyphenols for ER staining are marked with *.

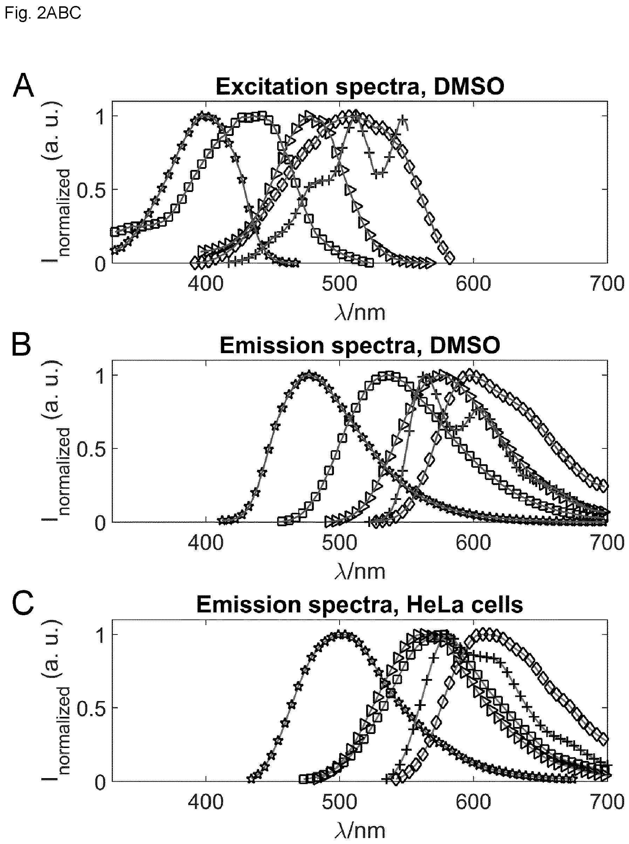

**Fig. 2****. Normalized fluorescence spectra of PB-P complexes.** Excitation **(A)** and emission **(B)** spectra in DMSO. **(C)** Cellular emission spectra as determined from microscopic images of the ER stained with **PB-6,** "stars"; **PB-1,** "squares"; **PB-5,** "triangles"; **PB-8,** "diamonds"; **PB-7**, "+" (2 peaks).

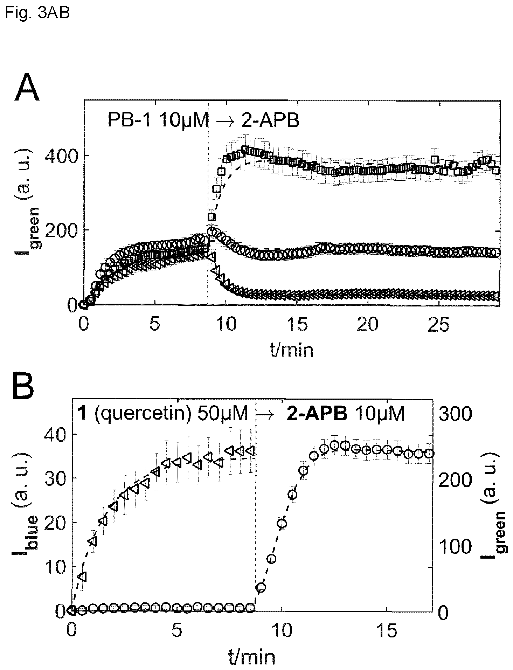

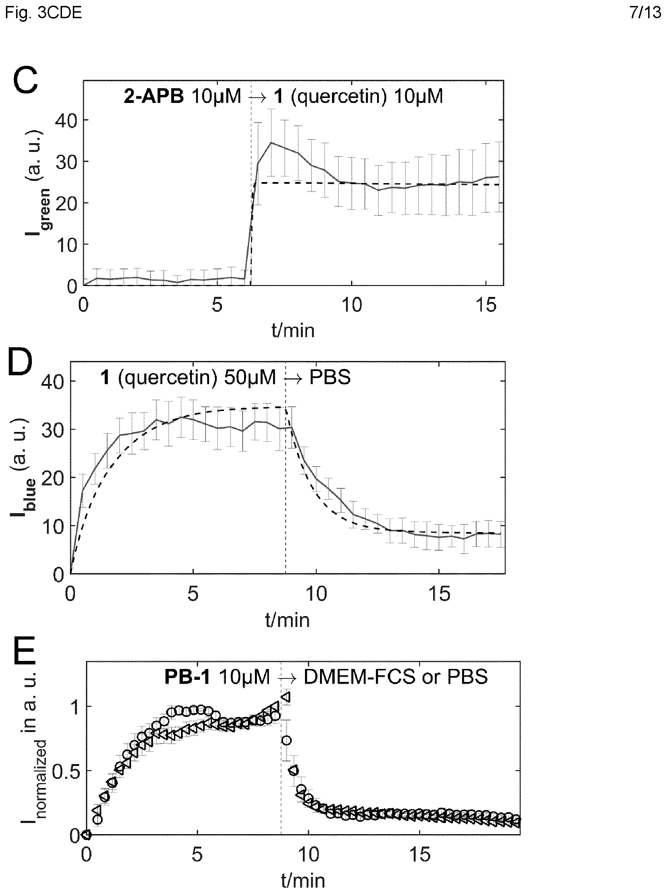

**Fig. 3****. Time-dependent enrichment and depletion dynamics of PB-1 in HeLa cells. (A)** The **PB-1** staining buffer was replaced by PBS containing 2-APB (1 µmol L⁻¹, "triangles"; 10 µmol L⁻¹, "circles"; and 50 µmol L⁻¹, "squares"), once the fluorescence intensity had reached a plateau (minute 8). Dashed black lines indicate model fits to the three datasets. **(B)** Intracellular fluorescence of 50 µmol L⁻¹ **1** (I_{blue}, "triangles") and **PB-1** (I_{green}, "circles") after washing with 2-APB (10 µmol L⁻¹). The dashed black line indicates the model fit. **(C) PB-1** fluorescence (I_{green}, gray solid line) due to sequential treatment with 2-APB (10 µmol L⁻¹) and **1** (10 µmol L⁻¹). The dashed black line indicates the model fit. **(D)** In-cell fluorescence intensity of **1** (50 µmol L⁻¹, gray solid line) reaches a residual level after exchange for PBS. The dashed black line indicates the model fit. **(E) PB-1** fluorescence rapidly declines after an exchange for growth medium (DMEM-FCS, "triangles") or for PBS ("circles") with an average half-life of 0.35 min. **(F)** Illustration of the ODE model used to simultaneously fit the experimental data shown in (A-D). The kinetic model describes the dynamic exchange of **1** (Q, quercetin), 2-APB (N, Neu's reagent), and **PB-1** (QN) between medium (Qₘ, Nₘ, QNₘ) and cytoplasm (Q_{c}, N_{c}, QN_{c}). Q_{c}, N_{c} and QN_{c} are reversibly recruited to the ER by association with binding sites for **1** (ER_{Q}) or 2-APB (ER_{N}) resulting in Q_{ER}, N_{ER} and QN_{ER}. QN_{c} can associate with ER_{Q} or ER_{N}, resulting in QN:ER_{Q} or QN:ER_{N}. I_{blue} measurements, were fitted by the sum of the model species Q_{c}+QN_{c}+Q_{ER}+QN_{ER}+QN:ER_{Q}+QN:ER_{N}, whereas, I_{green} measurements were fitted by the sum of model species QN_{ER}+QN:ER_{Q}+QN:ER_{N}. Intensity trajectories in (A) to (E) reflect averages from n=7 cells.
**Fig. 4****. Enhancement of PB-1 fluorescence intensity in the presence of protein (BSA) and pH- and solvent-dependent optical properties. (A)** Fluorescence excitation spectra (dashed lines) and emission spectra (solid lines) of probe **1** ("triangles"), **1** + 1 µmol L⁻¹ BSA ("circles"), and **PB-1** ("squares"), 20 µmol L⁻¹ each in PBS buffer at pH 7.4. **(B)** 18-fold fluorescence enhancement of **PB-1** complexes in the presence of BSA at molar ratios. Note the different y-scaling compared to (A). For illustration purposes, spectra of panel (A) are also shown to scale in (B) (excitation spectra, dashed lines; emission spectra, solid lines; **PB-1,** squares; **PB-1**+BSA). **(C)** Fluorescence emission spectra of **PB-1** (20 µmol L⁻¹) as a function of pH 6.6-8.5, without (top) and in the presence of 1 µmol L⁻¹ BSA (bottom). **(D)** Fluorescence spectra of **PB-1** (20 µmol L⁻¹) in dichloromethane (DCM, "x"), dimethyl sulfoxide (DMSO, "triangles"), acetonitrile (AcN, "circles"), methanol (MeOH, "squares"), and H₂O ("+"), normalized to the spectrum with the highest fluorescence intensity. Fluorescence is highest in AcN, then DCM and DMSO (DCM with maximum at lower wavelength than DMSO), and much lower in MeOH and lowest in H₂O.
**Fig. 5****. Fluorescent labeling of the endoplasmic reticulum of HEK cells with PB-1.** Calnexin-mCherry transfected HEK 293T cells were treated with **PB-1** and imaged as described in the experimental section. A subsection of cells expressed Calnexin-mCherry, whereas all cells were stained by **PB-1** (scale bar: 10 µm).
**Fig. 6****. Normalized fluorescence spectra of PB-P complexes in HeLa cells.** Cellular emission spectra as determined from microscopic images of the ER stained with **PB-P.** Order of maxima from lowest to highest wavelength: **PB-2, PB-6, PB-4, PB-3, PB-10, PB-5, PB-9, PB-1, PB-7, PB-8.**

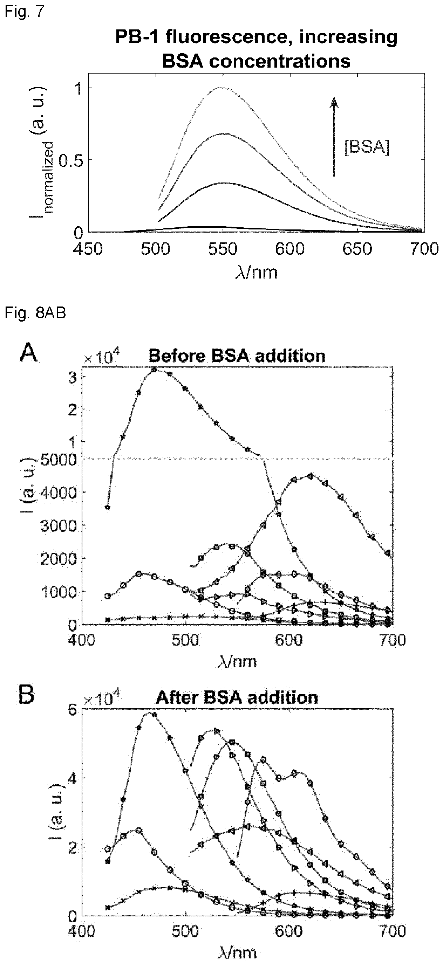

**Fig. 7****. Fluorescence spectra of 20 µmol L⁻¹ PB-1 in PBS as a function of bovine serum albumin (BSA) content in the buffer.** The maximum of the fluorescence emission is shifted from λₑₘ=537 nm in the absence (bottom line), to λₑₘ=550 nm in the presence of BSA (top lines). The fluorescent probe showed a fluorescence enhancement effect, which follows a linear relationship with increasing BSA concentration (1 µmol L⁻¹ increments). Further titration of BSA resulted in detector saturation.
**Fig. 8****.** Fluorescence spectra of 10 µM **PB-2** (piceatannol, "circles"), **PB-4** (rosmarinic acid, "x"), **PB-6** (esculetin, "stars"), **PB-3** (curcumin, "◄"), **PB-5** (luteolin, "►"), **PB-1** (quercetin, "squares"), **PB-7** (purpurin, "diamonds"), and **PB-8** (alizarin, "+") in PBS, before (A) and after (B) the treatment with equimolar concentrations of BSA. The fluorescence enhancement ratios strongly depended on the polyphenol, ranging from 2- to 60-fold at a 1:1 stoichiometry for **PB-P**:BSA.
**Fig**. **9****. Use of a polyphenol and 2-APB for determining the presence of a protein.** 100 µM solutions of the exemplary polyphenols rosmarinic acid and quercetin were each mixed with 2-APB (100 µM), and one of the proteins BSA (bovine serum albumin, 1 mM equivalent to 66 mg mL⁻¹), insulin (1 mg mL⁻¹), immunoglobulin IgG (0.05 mg mL⁻¹) in a total volume of 100 µl PBS. Samples were illuminated by an ultraviolet lamp.
**Fig. 10****. Use of PB-P complexes and BSA as fluorescent paints.** The photographed picture was painted on a glass plate with fluorescent colors, which consist of a polyphenol (piceatannol, esculetin, rosmarinic acid, kaempferol, or purpurin), the diaryl borinic compound 2-APB, and BSA. The plate was illuminated by an ultraviolet lamp, resulting in multiple color emission from blue (piceatannol), cyan (esculetin, rosmarinic acid), green (kaempferol), to orange (purpurin). Notably, intense fluorescence remained after the paints had dried.

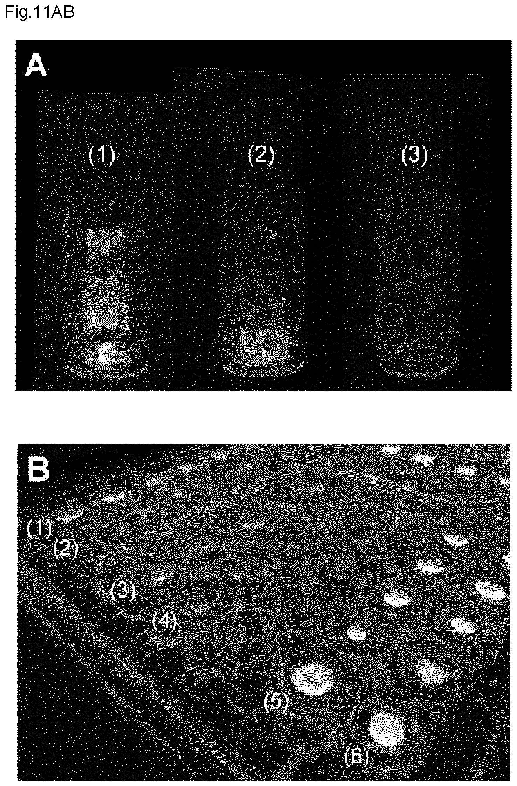

**Fig. 11****. (A) Crystals grown using the vapor diffusion method.** The depicted crystals were grown from mixtures of 2-APB and the polyphenols caffeic acid (vial 1), chlorogenic acid (vial 2), and isoquercitrin (vial 3). As can be seen, crystals are highly fluorescent, when illuminated at λₑₓ=365 nm. **(B)** Crystal growing using the hanging drop format. The depicted fluorescent droplets hanging from the coverslips comprise 2-APB and the polyphenols quercetin (row 1), rutin (row 2), isoquercitrin (row 3), epigallocatechine gallate (row 4), chlorogenic acid (row 5), and caffeic acid (row 6) dissolved in MeOH. In the picture shown, crystals were about to form. The 96-well plate was illuminated at λₑₓ=365 nm, demonstrating the intense fluorescence of the complexes in the dissolved and the crystalline phase.

### Examples

### Results and Discussion

Although potential cellular targets have been discussed for the chemopreventive effects of polyphenols, their definite biological mode of action is still obscure. This is in part due to the low bioavailability of polyphenols that interact with proteins and rapidly metabolize within the intestine.

The inventors therefore aimed to microscopically visualize the cellular uptake of autofluorescent 3,3',4',5,7-pentahydroxyflavone (quercetin, **1**), one of the most common dietary flavonoid polyphenolic compounds present in onions and apples. As determined by confocal live-cell imaging, **1** was rapidly internalized in HeLa cells, which was used as a cellular model for human epithelial tissue, and a faint fluorescence of the cytoplasm (*λ*ₘₐₓ=547 nm) was observed. To further improve the detection of **1**, the cells were subsequently exposed to 2-aminoethyl diphenylborinate (2-APB, Neu's reagent), an established reagent to determine flavonoids in plant tissues (Filippi et al., 2015, FEBS Open Bio 5: 748-752) and thinlayer chromatography (Wagner el. al. 1984. Plant Drug Analysis. A Thin Layer Chromatography Atlas Springer Verlag, Berlin, Heidelberg). 2-APB forms complexes with **1** under the loss of the ethanolamine chain, as confirmed by NMR spectroscopy and high resolution mass spectrometry. ¹H-NMR signals of the complex PB-1 (i.e., a complex of the phenyl-borinic compound 2-APB) and compound 1, quercetin) were significantly upfield-shifted (data not shown) and substantial variations of Δδ revealed the 3', 4'-o-diphenolic moiety at the B-ring as the main coordination site for the Lewis acidic organoboron compound (data not shown). In excess of 2-APB, bis-adducts were additionally formed, as previously shown for quercetin-3-O-glucorhamnosid (P. Matteini, et al., Monatsh. Chem. 2011, 142, 885). After incubation with 2-APB, a highly fluorescent filamentous network became visible within the quercetin-pretreated cells, consisting of tubular structures and cisterna (Fig. 1A), instead of the expected pervasive staining of the cytoplasm. Applying both dye components successively or as a premixed dye caused identical staining patterns. Confocal images and correlation analysis revealed that the space-resolved fluorescence of intracellular **PB-1** and the transiently expressed ER marker calnexin-mCherry follow the same spatial pattern (Fig. 1B), indicating the distinct labeling of the ER by **PB-1** in Hela as well as in HEK cells (Fig. 5).

The inventors next set out to investigate whether other natural products that form highly fluorescent complexes with 2-APB were capable of serving as fluorescent tags in live-cell imaging. Incubating the cells with the 2-APB complex of the catechol-bearing stilbene 3',4',3,5-tetrahydroxy-*trans*-stilbene (piceatannol, **2**) indeed resulted in a distinct ER staining. However, after an initial intracellular fluorescent burst, a significant drop in fluorescence intensity occurred, presumably due to light-induced isomerization to the cis-stilbene and irreversible 2,2'-cyclization, making **PB-2** not an ideal ER stain. 2-APB complexes of diferuloylmethane (curcumin, **3**) - featuring a keto-enol coordination site, but lacking a catechol moiety - and caffeic acid ester of 3-(3,4-dihydroxyphenyl)lactic acid (rosmarinic acid, **4**) - having catechol coordination sites - showed bright fluorescence in aprotic solvents (Scheme 1), but only weak cell staining.

In contrast, 3',4',5,7-tetrahydroxyflavone (luteolin, **5**), 6,7-dihydroxycoumarin (esculetin, **6**), and the hydroxyanthraquinones 1,2,4-trihydroxyanthraquinone (purpurin, **7**) and 1,2-dihydroxyanthraquinone (alizarin, **8**) proved to form highly emissive fluorescent complexes with 2-APB and distinctly stained the ER comparable to probe **1** but in different colors (molecular structures in Fig. 1F).

To derive structure-effect relationships, analogs of **1,** the 3,3',4',5,5',7-hexahydroxyflavone (myricetin, **9**) and 3,5,7,4'-tetrahydroxyflavone (kaempferol, **10),** were further tested as potential organelle probes. Interestingly, an additional OH-group at the B-ring of the flavonoid skeleton as with **9** resulted in highly fluorescent complexes with 2-APB in aprotic solvents, but caused weaker intracellular fluorescence compared to **PB-1.** Likewise, probe **10**, lacking an OH-group at the B-ring in comparison to **1**, showed weak intracellular fluorescence with 2-APB. In conclusion, the inventors reasoned that for bright fluorescent complexes of polyphenol and diphenyl borinic compound, either a pyrogallol, a catechol, or a keto-enol moiety next to a π-conjugated scaffold is important, whereas a catechol function plays a role for acting as a distinct ER probe.

The complexes of the diaryl borinic agent with catechins, such as epigallocatechin gallate (**PB-11,** λₑₓ=320 nm), hydroxybenzoic acids, and hydroxycinnamic acids, such as caffeic acid (**PB-12,** λₑₓ=345 nm) and its derivatives, as for instance chlorogenic acid (λₑₓ=405 nm), revealed to be highly fluorescent outside of cells. The fluorescence of the two-component dyes could be further enhanced in combination with proteins such as BSA or aprotic solvents. However, these polyphenols are less relevant for cell staining as they show no intracellular fluorescence or have low excitation wavelengths in the UV range.

The inventors further compared the emission spectra recorded in DMSO with the in-cell fluorescence spectra determined from microscopic images (Fig. 2BC, Fig. 6). Excitation and emission maxima (λₘₐₓ) of **PB-P** complexes in DMSO and HeLa cells are provided in Table 1 below. Cellular emission spectra were determined from microscopic images of the ER stained with **PB-P.**

**Table 1**

| complex | *λ*ₘₐₓ (nm) | | |
|---|---|---|---|
| | excitation (DMSO) | emission (DMSO) | emission (cellular) |
| **PB-1** | 442 | 536 | 574 |
| **PB-5** | 478 | 576 | 564 |
| **PB-6** | 399 | 477 | 502 |
| **PB-7** | 512 | 564 | 585 |
| **PB-8** | 513 | 597 | 613 |

The maxima of the cellular emission spectra were on average 25 (±9) nm bathochromically shifted as compared to the spectra in DMSO, but otherwise followed a similar trend, except for **PB-5** that underwent a blue-shift. **PB-5** may interact with the ER in a substantially different manner than the other probes. Most notably, the fluorescence spectra of the dyes cover a broad spectral range. The 2-APB complexes of **1**, **5**, **6**, **7**, and **8**, are particularly suited for fluorescence microscopy because they are easily excitable with the common laser wavelengths 405 (**PB-6**), 488 (**PB-1, PB-5**), and 561 nm (**PB-7, PB-8**).

Interestingly, washing stained cells with growth medium (e.g., DMEM-FCS) or phosphate buffered saline (PBS) lacking 2-APB, extinguished the fluorescence completely (Fig. 1A, wash). Adding 2-APB again shortly after the washing step, in turn, re-stained the ER, whereas a delayed supply (>10 min.) with 2-APB was not able to recover the fluorescence. These findings indicate that the polyphenol rapidly exits the cells, unless it is restrained by 2-APB. Next, the experiment was repeated with paraformaldehyde-fixed HeLa cells. The observation that fixed cells were successfully stained indicates passive diffusion of the small-sized dye molecules across the cell membrane rather than an active transport mechanism, which would be only effective in living cells.

For studying the cellular uptake dynamics of the fluorescent dyes, several live-cell imaging experiments were performed using exemplary the fluorescent complex **PB-1**, in combination with mathematical modeling. Fluorescence signals of **1** and **PB-1** were segmented for individual cells from microscopic images. The degree of staining revealed to be highly sensitive to the content of 2-APB in the washing solution (Fig. 3A). In principle, microscopy could be performed without washing off the staining solution, as the dye is only weakly fluorescent in aqueous environments. Nevertheless, best microscopic results were achieved with enhanced imaging contrast and a robust ER staining when the stained cells were washed with PBS containing 2-APB, e.g., about 10 µmol L⁻¹ 2-APB, which is non-fluorescent itself. Staining was also achieved by applying 1 and 2-APB sequentially (Fig. 3B and 3C). After removing compound **1**, the signal did not fully decay, indicating the presence of two compartments with different enrichment dynamics (Fig. 3D). In contrast, the signal of **PB-1** rapidly decayed (*t*_{1/2} ≈ 0.3 min) after washing with pure growth medium or PBS buffer (Fig. 3E).

To quantitatively describe the observed dynamics of dye loading and depletion, a mathematical model consisting of coupled ordinary differential equations (ODEs) was developed and fitted to the recorded dataset (Fig. 3). Essentially, systematic model refinement showed that two intracellular compartments, the cytosol and the ER, must be considered to explain the experimentally obtained dataset. Almost all model parameters were identifiable within reasonable confidence intervals (data not shown). Parameter estimates indicated that **1**, 2-APB and **PB-1** are enriched in the cells by several 1000-fold compared to the cell culture medium. Model discrimination indicated that binding of **1** and 2-APB to the ER is limited, although parameter estimation revealed that binding sites are highly abundant. Due to the pronounced accumulation of **1** and **PB-1** in the cytoplasm, both compounds are effectively bound despite their lower estimated affinities to the ER compared to 2-APB. **1** is slowly released from its ER-binding sites. This allows for stable labelling of the ER with **PB-1**, as long as 2-APB is sufficiently available in the cell culture medium. In contrast, the rapid accumulation and depletion of 2-APB makes a transient staining of the ER possible with the two-component dye. Exchange of 2-APB between medium, cytoplasm, and ER is fast and can thus be regarded as quasi-steady state. Depending on whether or not 2-APB is present, the ER fluorescence is switched on or off, enabling reversible chemical labeling (Fig. 1A).

The enormous intracellular fluorescent light-up may be attributed to the association of the probe with the ER or to its partitioning into the cholesterol-poor ER membranes. (Teraski, et al., CELL 1984, 38, 101). 2-APB inhibits inositol trisphosphate receptors (IP₃Rs) at the ER, as well as sarco/endoplasmic reticulum Ca²⁺-ATPase (SERCA) (Diver, et al. 2001, Cell Calcium 30, 323). Without intending to be bound by the theory, possibly, the organelle-selective accumulation of **PB-P** is based on the combination of a specific recognition of both dye components at the ER. In order to test the hypothesis whether the dramatically enhanced luminescence of intracellular **PB-P** was triggered by protein interactions of the dyes, we compared the fluorescence spectra of **PB-1** in PBS buffer with and without bovine serum albumin (BSA) (Fig. 4AB). BSA is known to bind to polyphenols (Skrt et al., 2012, Food Chem. 135, 2418; Mishra et al. 2005, J. Chem. Sci. 117, 641).

Upon the addition of BSA, the inventors observed a remarkable enhancement of the fluorescence intensity in comparison to the aqueous staining solutions. The fluorescence signal linearly increased with the protein concentration and easily exceeded the range of the detector (Fig. 7). This effect could be confirmed with other proteins tested, e.g., insulin, immunoglobulin G or egg white (comprising 54% ovalbumin, 12% ovotransferrin, 11 % ovomucoid and several other proteins) and human saliva (data not shown).

The inventors found that all ER stains tested underwent a further fluorescence enhancement effect with various degrees when BSA was added (e.g. 2- to 60-fold at a 1:1 stoichiometry for **PB-P**:BSA) (Fig. 8AB), The effect was much stronger than with lysozyme, which is known to only weakly bind to polyphenols (Rawel et al. 2006, Mol. Nutr. Food Res. 50, 705), data not shown.

The influence of the pH on the fluorescence intensity was assessed by gradually titrating (pH 6.6-8.5) **PB-1** with an increasing amount of NaOH. Under the more alkaline conditions that induce further deprotonation of the hydroxyl groups of the flavonol (Momicet al. 2007, Collect. Czechoslov. Chem. Commun. 72, 1447), the fluorescence intensity was shown to be lower, and the emission spectrum bathochromically shifted (Fig. 4C, top). In the presence of BSA, the location of the fluorescence maximum was rather independent from the pH of the solution, but the fluorescence intensity was significantly more sensitive to the solution pH than without protein (Fig. 4C, bottom), suggesting that pH-induced conformational changes of the protein cause the strong pH dependence of the fluorescence strength of the ternary system. As the fluorescence intensity was correlated with the pH of the solution, combinations of **PB-1** and BSA are applicable as pH indicator.

To further elucidate the photophysics behind the amplified fluorescence, the inventors studied solvent effects on the fluorescence properties of **PB-1** and observed a pronounced change in the fluorescence intensity depending on the proton-donating ability of the solvent. Whereas the fluorescence intensity was found to be extraordinarily high in the aprotic solvents DMSO, dichloromethane, and particularly in acetonitrile, fluorescence dropped in protic solvents, such as MeOH or water (Fig. 4D). **1** is considered a prototype compound for excited-state intramolecular proton transfer (ESIPT) (Simkovitch, et al. 2015, J. Phys. Chem. B 119, 10244), a photochemical process that generates a keto-enol tautomer with different electronic structure compared to the initial excited form. In aprotic solvents, the fluorescence of **PB-1** may exclusively result from the phototautomer T* (ESIPT emission band), whereas in polar protic solvents, the intramolecular hydrogen bond is replaced by intermolecular H-bridges with solvent molecules, accelerating the non-radiative relaxation of the excited fluorophores (Das et al., 2009, Photochem. Photobiol. Sci. 8, 1583; Santos et al. 2014, Photochem. Photobiol. Sci. 13, 992). The inclusion of the complex into a protein binding pocket may in turn retain the ESIPT emission as the binding protein shields the complex from H-donating solvent molecules similar to cyclodextrins (Das et al., 2009; Zhuang et al. 2013, Angew. Chem. Int. Ed. 52, 8124; Suzuki et al. 2014, Angew. Chem. Int. Ed. 53, 8231). In accordance, **PB-5** and **PB-7** (Peng et al. 2017, J. Phys. Chem. A 121, 5625) bear tautomeric systems capable of forming an intramolecular hydrogen bond and have extraordinarily high turn-on ratios in the presence of BSA (Fig. 8B).

In summary, the inventors described a variety of natural fluorophores that form environment-sensitive, highly fluorescent complexes with 2-APB. The dyes feature relatively large Stokes shifts, protein and solvent specific fluorescence enhancement, the facility of structural modification, and therefore a broad range of emission wavelengths. These characteristics open up new opportunities for use of novel fluorogenic probes desirable for biochemical studies, bioimaging, and microenvironment sensing, such as selective protein detection.

The results prove that, particularly, the fluorescent 2-APB complexes of the polyphenols **1**, **5**, **6**, **7**, and 8 are suited for reversibly labeling the ER of living and fixed cells, which was confirmed with the transiently expressed ER markers calnexin-mCherry and calnexin-mGFP. The chemical staining method with **PB-P** enables a convenient, versatile ER labeling, e.g., in fixed samples and non-transfectable cells by simply adding the probe and incubating briefly. Subsequent washing off the staining solution with 2-APB containing buffer or growth medium is not mandatory, but recommended to keep the background fluorescence low. If required, the ER fluorescence can be easily *erased* by thoroughly removing 2-APB from the cellular environment. 2-APB conjugates are thus cheap alternatives to ER-Tracker™ dyes. The ease of dye preparation, the fast (de)staining kinetics, and the cost-effectiveness make the new polyphenol-based fluorescent dyes, which the inventors name *ERasers,* especially attractive chemical ER tags.

### Experimental Procedures

### Cell Culture

HeLa (human cervix carcinoma) and HEK 293T (human embryonic kidney) cells were maintained in DMEM (Invitrogen, Darmstadt, Germany) supplemented with 10% fetal calf serum (Biochrom AG, Berlin, Germany), 100 µg mL⁻¹ penicillin and streptomycin (Invitrogen) in a humidified atmosphere of 5% CO₂ at 37°C. Cells were passaged routinely upon reaching 80% confluence and were used for the assays during passages 5-15. For fluorescence microscopy, cells were maintained in 8-well Lab-Tek chambers (Thermo Scientific, Asheville, NC, USA) with a density of 40.000/well, which were kept in an incubation chamber at 37 °C enriched with 5% CO₂. Both cell types were transfected with the Calnexin-mCherry and Calnexin-mGFP fusion constructs, which were a kind gift of Joel Beaudouin and constructed as described in Beaudouin et al.,^{[1]} using X-tremeGENE 9 (Roche Pharma, Basel, Switzerland). Cells were fixed with 4% paraformaldehyde (Sigma-Aldrich, Steinheim, Germany) in phosphate buffered saline (PBS) for 10 minutes.

### Reagents

Concentrated stock solutions (20 mmol L⁻¹) of the complexation reagent 2-aminoethyl diphenylborinate (2-APB) (Sigma-Aldrich, Steinheim, Germany) and of the polyphenols kaempferol (**10**), luteolin (**5**), and isoquercitrin (Extrasynthese, Genay, France) quercetin (**1**), curcumin (**3**), rosmarinic acid (**4**), and purpurin (**7**), epigallocatechin gallate (**11**), caffeic acid (**12**) (Sigma-Aldrich, Steinheim, Germany), esculetin (**6**), alizarin (**8**), myricetin (**9**), rutin, and chlorogenic acid (Carl Roth, Karlsruhe, Germany), piceatannol (**2**) (TCI Europe, Zwijndrecht, Belgium) were prepared in absolute DMSO (Merck, Darmstadt, Germany). The fluorescent compounds **PB-1-PB-12** were synthesized by mixing the polyphenol stocks **1-12** with 2-APB in a 1:1 molar ratio. The DMSO stock solutions were stored at - 20°C until further use. All commercial chemicals were of analytical grade or better and were used as such without further purification.

### Cellular Staining Protocol

For cell staining experiments, **PB-1-PB-10, 1-10,** and 2-APB were freshly diluted with PBS (or DMEM). Cells were treated with 10 µmol L⁻¹ **PB-1-PB-10** for 8 minutes, which gave optimal staining results. The staining solution was then gently pipetted off and immediately replaced by 10 µmol L⁻¹ 2-APB. For sequential staining experiments, the cells were first treated with 10 µmol L⁻¹ polyphenol **1-10** for 8 min. The polyphenol solution was then gently removed and immediately replaced by 10 µmol L⁻¹ 2-APB. Control samples were incubated with equivalent concentrations of DMSO and 2-APB, respectively. DMSO in the bath solution did not exceed 0.1%.

### Confocal Microscopy and Image Processing

In order to determine optimal filter sets for imaging of the stained cells, emission spectra of fixed HeLa cells, treated with **1** or **PB-1-PB-10,** were recorded using a Leica SP5 confocal laser scanning microscope (Leica Microsystems CMS GmbH, Mannheim, Germany), equipped with an acousto-optical beam splitter (AOBS). **1**, **PB-2**, **PB-4**, and **PB-6** were excited with a diode laser at *λ*ₑₓ=405 nm, **PB-3** with the *λ*ₑₓ=458 nm line of an argon laser, **PB-1**, **PB-5**, **PB-9**, and **PB-10** with the *λ*ₑₓ=488 nm line of the argon laser, and **PB-7** and **PB-8** were excited at *λ*ₑₓ=561 nm with a solid state laser. Spectral image sets were recorded between 385 and 775 nm by restricting emission filters to intervals of 10 nm. Averages were taken from n=35 cells for each **PB-P** complex (7 cells from 5 positions, respectively). Live-cell experiments were performed on a Nikon Ti inverted microscope, equipped with a CSU-22 Yokogawa confocal spinning disc slider (Yokogawa Electric Corporation, Tokyo, Japan), a 60x Plan Apo NA 1.4 objective lens (Nikon, Tokio, Japan), a Hamamatsu C9100-02 EMCCD camera (Hamamatsu Photonics, Hamamatsu, Japan), and the Volocity software (PerkinElmer). The intracellular fluorescence (I_{blue}) of **1** and **PB-6** were excited at λₑₓ=405 nm and emission was collected using a 445/60 emission filter (Chroma Technology Corp). Intracellular green fluorescence of **PB-1** (I_{green}) and mGFP fluorescence were excited at *λ*ₑₓ=488 nm and emission was collected through a 527/55 emission filter (Chroma Technology Corp, Bellows Falls, VT, USA). **PB-7** and mCherry fluorescence were excited at *λ*ₑₓ=561 nm and their red emission was collected with a 615/70 filter (Chroma Technology Corp). For time-resolved experiments, images were recorded at a time interval of 30 seconds. All microscopic images were evaluated with ImageJ software (NIH, Bethesda, MA, USA).

### Mathematical Modeling and Statistical Methods

Model simulations were performed with custom scripts in MATLAB (The Mathworks, Natick, MA, USA). For parameter calibrations, all ODE models were implemented with the MATLAB toolbox PottersWheel (http://www.potterswheel.de). As a measure for the goodness of fit the Akaike information criterion (AIC) was used.

### Fluorescence Spectroscopy

Fluorescence excitation and emission spectra were recorded using a standard 1 cm quartz cuvette on a Cary Eclipse fluorescence spectrophotometer (Agilent Technologies, Wald-bronn, Germany) with a bandpass filter of 5 nm for both excitation and emission. 1 mmol L⁻¹ NaOH (Merck, Darmstadt, Germany) was used to adjust the pH value, in pH titration studies. PBS, bovine serum albumin (BSA), insulin from bovine pancreas, lysozyme were obtained from Sigma-Aldrich (Steinheim, Germany), and IgG from Invitrogen. The solvents acetonitrile, dichloromethane, and methanol were purchased from J.T. Baker (Deventer, Netherlands). Ultrapure water was prepared with a Milli-Q Gradient A10 system (Millipore, Schwalbach, Germany).

### Fluorescent Paints

Mixtures of polyphenols, 2-APB, and proteins can be used as fluorescent paints. In this application example 100 µM of either piceatannol, esculetin, rosmarinic acid, kaempferol, or purpurin were each mixed with 2-APB (100 µM) and BSA (1 mM) in PBS. The fluorescent colors were used for painting on a glass plate, which was later illuminated by ultraviolet light.

### Fluorescent Crystals

Crystals were prepared by providing a supersaturated solution of 2-APB and the respective polyphenol until crystallization set in. Supersaturation was achieved by the vapor diffusion method using two vials, one of which fitted into the other. In the inner vial, 500 mM 2-APB and 33-500 mM of an exemplary polyphenol (quercetin, rutin, isoquercitrin, epigallocatechine gallate, chlorogenic acid, and caffeic acid) were dissolved in MeOH. Diethyl ether was added to the second vial and the entire system was sealed with a lid and parafilm. While diethyl ether slowly diffused into the inner vial, crystals formed. Alternatively, the hanging drop format was employed to grow crystals. For this purpose, 2 µl droplets of the 33-500 mM methanolic solutions to be crystallized were pipetted onto coverslips. These were attached upside down on a crystallization tray, which was filled in the reservoir with same solution in higher concentration. The system was sealed with silicone oil that equilibrium between the drop and the reservoir could be achieved. Crystallization trays were stored at room temperature.

### NMR Spectroscopy

1D- and 2D-NMR spectra (¹H; ¹³C; ¹H-¹H-COSY; heteronuclear single-quantum coherence, HSQC; heteronuclear multiple-bond correlation, HMBC) of **PB-1-PB-12, 1-12,** and 2-APB (5 mmol L⁻¹ in DMSO-de) were acquired on an Avance III 500 MHz spectrometer with a cryo-TCI probe and an Avance III 400 MHz spectrometer with a BBO probe (Bruker, Rheinstetten, Germany) using Bruker TopSpin™ 3.0 software. In order to spectroscopically monitor the formation of **PB-1,** 2-APB was incrementally titrated to 1 (10 mmol L⁻¹) to obtain the ratios **1** (1): 2-APB (0.1-10). Data was processed using MestReNova software (Bruker), referenced to the solvent DMSO-*d₆* (Euriso-top, Gif-sur-Yvette, France), and reported in *δ* (ppm). Coupling constants *J* were listed in Hz.

### Mass Spectrometry

High resolution mass spectra (HRMS) of **PB-1-PB-12** were acquired with a Waters Synapt G2-Si HDMS mass spectrometer (Waters, Manchester, UK), operating in negative ionization mode. The DMSO stock solutions of **PB-1-PB-12**, **1-12**, and 2-APB were diluted with MeOH to obtain a final concentration of 10 µmol L⁻¹ and directly infused into the ESI-source at a flow rate of 10 µL min⁻¹.

## Claims

1. A use of a polyphenol and a diaryl borinic compound in combination with an agent capable of providing an aprotic environment selected from the group consisting of the endoplasmic reticulum (ER) of an animal cell, a protein, and a solid phase of a complex formed by the polyphenol and the diaryl borinic compound,
for providing an increased fluorescence, wherein the fluorescence is increased by a factor of at least 2 in the presence of the agent compared to the absence of the agent.

2. A use of a polyphenol and a diaryl borinic compound for selective fluorescence staining of the endoplasmic reticulum (ER) of an animal cell, wherein the use optionally is a use of claim 1.

3. A method for selective fluorescence staining of the endoplasmic reticulum (ER) of an animal cell, comprising contacting the cell with a polyphenol and a diaryl borinic compound, wherein the analysis is carried out in the presence of the diaryl borinic compound.

4. A use of a polyphenol, a diaryl borinic compound, and an agent capable of providing an aprotic environment, wherein the use optionally is a use of claim 1, for
(a) determining the proton-donating ability of a solvent,
preferably, for determining the pH of an aqueous composition, wherein the agent is a protein selected from the group comprising albumin;
(b) determining the presence and/or concentration of the agent in a composition, wherein the agent is selected from the group comprising a protein and an aprotic solvent, wherein the agent preferably is a protein;
(c) for determining the presence and/or concentration of the polyphenol;
(d) for determining the presence and/or concentration of the diaryl borinic compound; or
(e) for providing light in a light emitting device, wherein the agent preferably is a solid phase of a complex formed by the polyphenol and the diaryl borinic compound in the presence of a protein.

5. The use of claim 2 or the method of claim 3, wherein the staining of the ER is reversible, wherein the ER is stained in the presence of the diaryl borinic compound and the ER is not stained in the absence of the diaryl borinic compound.

6. The use of any of claims 2 or 5 or the method of any of claims 3 or 5, wherein the staining of the ER is performed by contacting the cells with the polyphenol, preferably, with a mixture of the polyphenol and the diaryl borinic compound, and then washing the cells with the diaryl borinic compound, wherein the staining is analysed in the presence of the diaryl borinic compound.

7. The use of any of claims 1-2 or 4-6 or the method of any of claims 3 or 5-6, wherein the diaryl borinic compound is a compound of formula 10, 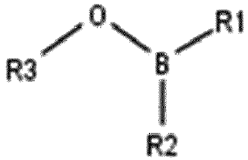 wherein R1 and R2 are independently from one another selected from non-substituted aryl, substituted aryl, non-substituted heteroaryl and substituted heteroaryl, preferably, non-substituted phenyl,
wherein R3 is C₂-C₁₀ alkylamino, C₁-C₁₀ alkyl, C₂-C₁₀ alkoxy, C₆-C₁₀ aryl, C₆-C₁₀ heteroaryl or C₇-C₁₀ aralkyl, preferably, C₂-C₁₀ alkylamino,
wherein, optionally, the diaryl borinic compound is 2-aminoethoxydiphenyl borate.

8. The use of any of claims 1-2 or 4-7 or the method of any of claims 3 or 5-7, wherein the polyphenol comprises a catechol, a pyrogallol and/or a keto-enol moiety or a derivative thereof, wherein the catechol, pyrogallol and/or keto-enol groups of said moieties are substituents of a π-conjugated scaffold comprising at least 6 delocalized electrons,
wherein, if the use is a use of claim 2 or if the method is a method of claim 3, the π-conjugated scaffold preferably comprises 9-16 delocalized electrons.

9. The use of any of claims 1-2 or 4-8 or the method of any of claims 3 or 5-8, wherein the polyphenol is
a) a flavonoid selected from the group comprising flavonole, hydroxyflavone, flavan-3-ol, anthocyanidine, flavanone, isoflavone,
b) a hydroxyantraquinone,
c) a benzopyrone,
d) a stilbenoid,
e) a chalcone,
f) a curcuminoid,
g) a hydroxycinnamic acid,
h) a hydroxybenzoic acid,
i) pyrocatechol,
or a derivative thereof, wherein the derivative preferably is an ether, a glycoside or an ester of the polyphenol.

10. The use of any of claims 1-2 or 4-9 or the method of any of claims 3 or 5-9, wherein the polyphenol is quercetin, piceatannol, curcumin, rosmarinic acid, luteolin, myricetin, esculetin, purpurin, alizarin, epigallocatechin gallate, caffeic acid, or a derivative thereof, wherein the derivative optionally is a glycoside,
wherein the polyphenol preferably is quercetin.

11. The use of any of claims 1-2 or 4-10 or the method of any of claims 3 or 5-10, wherein the π-conjugated scaffold of the polyphenol comprises two or three aromatic or heteroaromatic rings, wherein at least two of said rings are fused and wherein the polyphenol comprises two, but not three vicinal hydroxyl groups, wherein, preferably, the use is the use of claim 2.

12. The use of any of claims 1-2 or 4-11 or the method of any of claims 3 or 5-11, wherein the polyphenol and the diaryl borinic compound are capable of reacting to form a fluorogenic complex comprising the structural element of formula 11: 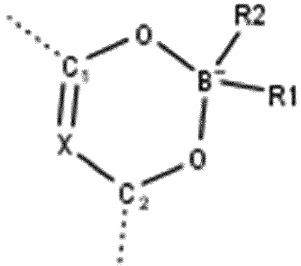 wherein R1 and R2 are independently selected from non-substituted aryl, substituted aryl, non-substituted heteroaryl and substituted heteroaryl, w
herein X is absent or present,
and wherein, if X is absent, the double bond indicated between C₁ and X is between C₁ and C₂,
and wherein, if X is present, X is C,
wherein C₁ and C₂ and, if present, X, are part of an aromatic or heteroaromatic system, which system may be substituted or non-substituted,
wherein, preferably, X is absent.

13. The use or the method of claim 12, wherein X is absent, and C₁ and C₂ are part of an aromatic or heteroaromatic ring system, which ring-system may be substituted or non-substituted (formula 11 a):

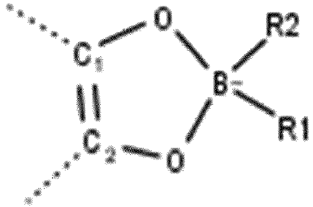

14. A kit suitable for the use of any of claims 1-2 or 3-13, wherein the kit comprises
(i) a composition comprising (a) a polyphenol and, preferably, (b) a diaryl borinic compound,
(ii) a composition comprising the diaryl borinic compound,
wherein the kit optionally further comprises (c) a protein selected from the group comprising albumin.

15. A kit of claim 14 comprising at least 2, preferably, 3 or 4 different polyphenols, each having, in complex with the diaryl borinic compound, an emission wavelength that can be differentiated from the emission wavelength of the other complex(es), wherein the kit optionally comprises a separate composition (i) for each polyphenol.

16. An isolated fluorescent complex comprising (a) a polyphenol, (b) a diaryl borinic compound, and (c) a protein selected from the group comprising albumin, or a kit comprising (a), (b) and (c), wherein the complex is preferably obtainable by combining the components of the kit of any of claims 14 or 15.

17. A kit, complex or composition, optionally, a kit of any of claims 14-15, or a complex of claim 16, comprising (a) a polyphenol that is not a flavonoid and (b) a diaryl borinic compound,
or use of said diaryl borinic compound for analysing the presence and/or concentration of said polyphenol, each, optionally, in the presence of (c) a protein selected from the group comprising albumin.

18. A solid, optionally, crystalline form of a complex of (a) a polyphenol and (b) a diaryl borinic compound, wherein the complex optionally further comprises (c) a protein selected from the group comprising albumin.
